# EUROPEAN PATENT SPECIFICATION

(11) **EP 4 552 588 B1**
(45) Date of publication and mention of the grant of the patent: **14.01.2026**
(21) Application number: 23383150.2
(22) Date of filing: 09.11.2023
(51) Int. Cl.: A61B 17/12, A61B 34/10, A61B 17/00

(54) **COMPUTER-IMPLEMENTED METHOD FOR THE IN SILICO SURGICAL PLANIFICATION OF A LEFT ATRIAL APPENDAGE OCCLUSION**
COMPUTERIMPLEMENTIERTES VERFAHREN ZUR CHIRURGISCHEN IN-SILICO PLANIFIKATION EINER OKKLUSION DES LINKEN HERZOHRES
PROCÉDÉ MIS EN UVRE PAR ORDINATEUR POUR LA PLANIFICATION CHIRURGICALE IN SILICO D'UNE OCCLUSION D'APPENDICE AURICULAIRE GAUCHE

(43) Date of publication of application: 14.05.2025
(73) Proprietor: Universitat Pompeu Fabra, 08002 Barcelona (ES); Hospital Clínic de Barcelona, 08036 Barcelona (ES); Fundació de Recerca Clínic Barcelona-Institut d'Investigacions Biomèdiques August Pi i Sunyer (FRCB-IDIBAPS), 08036 Barcelona (ES); Fundació Institut de Recerca de l'Hospital de la Santa Creu i Sant Pau, 08041 Barcelona (ES)
(72) Inventor: Cámara Rey, Oscar, 08002 Barcelona (ES); Mill Tena, Jordi, 08002 Barcelona (ES); Olivares, Andy Luis, 08002 Barcelona (ES); Aguado Martín, Ainhoa Marina, 08002 Barcelona (ES); Albors Lucas, Carlos, 08002 Barcelona (ES); Saiz Vivó, Marta, 08002 Barcelona (ES); Herrero Díaz, Ángel, 08002 Barcelona (ES); Arzamendi Aizpurua, Dabit, 08041 Barcelona (ES); Freixa Rofastes, Xavier, 08036 Barcelona (ES)
(74) Representative: Hoffmann Eitle

(56) References cited:
- US-A1- 2023 119 535
- PLANAS ERIC ET AL: "In-silico Analysis of Device-Related Thrombosis for Different Left Atrial Appendage Occluder Settings", 1 January 2022, 20220101, PAGE(S) 160 - 168, XP047618656
- MORALES XABIER ET AL: "Towards Real-Time Optimization of Left Atrial Appendage Occlusion Device Placement Through Physics-Informed Neural Networks", 28 January 2023, 20230128, PAGE(S) 36 - 45, XP047646960
- ALBORS CARLOS ET AL: "Impact of Blood Rheological Strategies on the Optimization of Patient-Specific LAAO Configurations for Thrombus Assessment", 16 June 2023, FUNCTIONAL IMAGING AND MODELING OF THE HEART; [LECTURE NOTES IN COMPUTER SCIENCE; LECT.NOTES COMPUTER], SPRINGER NATURE SWITZERLAND, CHAM, PAGE(S) 485 - 494, ISBN: 978-3-031-35301-7, ISSN: 0302-9743, XP047661146

## Description

### Technical field of the invention

The present invention relates to the field of medical technology and diagnostics, particularly relates to computer-assisted methods for cardiovascular surgical planification involving risk assessment. More specifically, the invention relates to a computer-implemented method for the *in silico* surgical planification of a left atrial appendage occlusion.

### Background of the invention

Atrial Fibrillation (AF) is the most prevalent cardiac arrhythmia in clinical practice, with a significant impact on public health due to its association with ischemic stroke. The left atrial appendage (LAA) is a common site for thrombus formation in AF, which can lead to stroke if a clot dislodges and travels to the brain. The complex anatomy of the LAA, which includes varying morphologies, complicates the management of stroke risk in AF patients.

When anticoagulation therapy is contraindicated or poses significant risks, clinicians may resort to left atrial appendage occlusion (LAAO) procedures. These interventions involve implanting devices like the commercially available Watchman, or the Amplatzer Amulet, via a transvenous approach, guided by imaging modalities such as fluoroscopy and echocardiography.

The success of LAAO depends on the accurate sizing and positioning of the device to ensure complete occlusion, necessitating a careful planification and a detailed analysis of the anatomical characteristics of the left atrium of the patient and the geometry and physical characteristics of the device. This planification process usually require intensive computing resources in order to perform the adequate simulations, which make it impractical to test different configurations. In other approach, this process is oversimplified in order to speed it up, however at the cost of failing to reproduce adequately the physical interaction between the LAA occluder and the left atrial appendage of the patient. Therefore, current planification processes are unable to provide an adequate LAAO surgical planification.

Furthermore, implantation of a left atrial appendage occlusion device carries an inherent risk of thrombus formation due to device-related factors. Incorrect device sizing, suboptimal placement, or inappropriate device morphology can lead to inadequate closure of the appendage and/or to blood flow disturbances such as recirculation and stagnation. These hemodynamic alterations around the device may, in turn, become sites for thrombus generation. Additionally, the material or the shape of the occluder can induce clot formation by activating a coagulation cascade. Therefore, precise device selection and accurate placement are critical to prevent thrombogenic complications post-implantation. Also, achieving a successful closure without residual flow into the LAA is crucial; inadequate occlusion may necessitate further intervention and poses a persistent risk for thrombus formation. All these aspects are currently not taken into consideration when performing a surgical planification for a LAAO, instead, simple geometrical relations are considered when choosing the position and the type of occluder for a LAAO surgery, leaving outside the steps of finding an optimal position for the device that also minimizes the risk of generating associated haemodynamic complications.

Current attempts to address these issues frequently rely on simplified blood flow velocity measurements, which do not adequately represent the complexities of atrial hemodynamics. For instance, state-of-the-art computational fluid dynamics (CFD) simulations take as input a limited number of parameters that do not characterize precisely, nor in a personalized and patient-specific way the blood flow in the left atrium of a patient. Similarly, the analysis of the imaging techniques to obtain a 3D model representation of the LAA of the patient are not exhaustive and leave out relevant features to properly assess the mechanical and haemodynamic features that are key in a LAAO surgical procedure planification. Therefore, these oversimplified and generic CFD simulations and imaging analysis do not allow to extract all the information needed to evaluate the most relevant parameters when assessing the durability of the device placement and/or the risk of device-associated thrombus formation for a specific patient. These limited and non-specific CFD simulations and 3D analysis, in turn, lead to a lack of output parameters that could become reliable indexes of thrombus formation and device safety.

Furthermore, the technical demands of LAAO also require advanced training and skill, underscoring a gap in the current approach, as there is an unmet need for computational tools that can provide a detailed, quick, objective, and patient-specific characterization of LAA morphology and its associated haemodynamics, to guide the selection and planning of LAAO interventions, taking associated risks into consideration such as the formation of LAAO associated blood cloths or the potential damage on the LAA walls due to the interaction with the occluder.

It is therefore needed a planification method for LAAO surgeries that take all these important aspects in consideration. That provides a quick, but most importantly, reliable estimate of an structurally safe position of the device that allows the clinician to evaluate the best scenarios, and which can also simulate precisely the blood flow, taking into account the most relevant parameters in order to be able to estimate an associated risk of thrombus generation.

US 2023/119535 A1 describes a computer implemented method according to the preamble of claim 1.

### Summary of the invention

A first aspect of the invention refers to a computer implemented method for *in silico* surgical planification of a left atrial appendage (20) occlusion (LAAO), the method comprising the steps of:
a. generating a 3D model of the left atrium (30) of a patient from medical images of a patient;
b. computing one or more inner diameters of the lumen of the left atrial appendage (LAA) (20), wherein said left atrial appendage (20) is comprised in the 3D model of the left atrium (30) of the patient generated in step a);
c. positioning a 3D model of a left atrial appendage occluder (10) at least partially inside the left atrial appendage (20), further wherein said position is determined according to one or more parameters selected from the list comprising: one or more diameters selected from the one or more diameters calculated in step b), a landing zone position comprised in the 3D model of the left atrial appendage (20), a first compression value of the left atrial appendage occluder (10), and anatomical characteristics extracted from the 3D model of the left atrium (30) of the patient, preferably wherein the anatomical characteristics comprise the shape and position of the ostium and/or the shape and position of the oval fossa;
d. performing a structural simulation to calculate a second compression value of the left atrial appendage occluder (10) from its interaction with the inner walls of the left atrial appendage (20);
e. if the calculated second compression value in step d) is within a range of acceptance, proceeding with step f), and if the second compression value calculated in step d) has a value outside said range of acceptance, continuing by going back to step c) and performing step c) again with one or more parameters modified, wherein said parameters are preferably the landing zone, the first compression value and/or the one or more diameters;
f. calculating a set of thrombosis risk factors using parameters derived from computational fluid dynamics (CFD) calculations and using medical parameters of the patient;
g. computing a risk score of developing thrombosis related to the left atrial appendage (20) occlusion positioning from step c), wherein said risk score is at least based on the thrombosis risk factors calculated in step f);

In a preferred embodiment of the method of the first aspect of the invention, if the risk score computed in step g) is above a risk threshold value, modifying the landing zone position, the first compression value and/or the one or more diameters selected from the one or more diameters calculated in step b), and performing step c) again.

In a preferred embodiment of the method of the invention, if the risk score computed in step g) is above a risk threshold value, displacing the 3D model of the left atrial appendage occluder (10) to a different position according to a database of CFD simulations performed on LAAO from other patients and repeating step d).

In a preferred embodiment of the method of the invention, the medical images of the patient are generated from one or more imaging techniques from the list comprising: ultrasound (US) imaging, Transthoracic Echocardiography (TTE), Computed Tomography (CT) imaging, Magnetic Resonance Imaging (MRI) imaging, 3D Rotational Angiography (3DRA) imaging, mitral valve pulsatile ultrasound imaging, and pulmonary veins pulsatile ultrasound imaging; preferably wherein the 3D model of the left atrium (30) of the patient is generated using input images generated using mitral valve pulsatile US imaging and either one of MRI, 3DRA or CT imaging; more preferably wherein he 3D model of the left atrium (30) of the patient is generated using input images generated using mitral valve pulsatile US, pulmonary valve pulsatile US, and either one of MRI, 3DRA or CT imaging.

In a preferred embodiment of the method of the invention, the first compression value is based on a volumetric relationship between the volume defined by the unmodified 3D model of the occluder (10) and the volume defined by the 3D model of the occluder (10) when its volume is modified to fit to the dimensions of the internal walls of the 3D model of the left atrium (30) of the patient.

In a preferred embodiment of the method of the invention, the position of the LAA occluder determined in step c) is further determined according to clinical data of the patient, preferably wherein the clinical data of the patient comprises one or more of the elements from the list comprising: CHADS2-VASc, HAS-Bleed and ultrasound data; more preferably the clinical data further comprises one or more elements from the list comprising: left atrium pressure, haemoglobin levels, BNP, C-Protein levels, Troponine levels, eGFR, ECG data, and creatinine levels; even more preferably wherein the clinical data of the patient comprises all the elements of both lists.

In a preferred embodiment of the method of the invention, the position of the LAA occluder determined in step c) is further determined according to at least one or more elements from the list comprising: a landing zone position comprised in the 3D model of the left atrial appendage (20), a database of left atrial appendage occluders (10) positioned in the left atrial appendage (20) of other patients, mechanic and volumetric characteristics of a catheter, wherein said catheter is associated to the positioning of the left atrial appendage occluder (10), and ultrasound images; preferably wherein the position of the LAA occluder is determined according to all the elements of said list.

In a preferred embodiment of the method of the invention, the medical parameters of the patient comprise CHADS2-VASc and/or HAS-Bleed.

In a preferred embodiment of the method of the invention, the thrombosis risk factors derived from CFD calculations comprise one or more of the elements from the list comprising: the average blood velocity in the pulmonary ridge area, the presence and average blood velocity of eddies, and/or stagnated flow in the pulmonary ridge area and device surface, the particle attachment quantity, and thrombogenic haemodynamic indexes; preferably wherein the thrombosis risk factors derived from CFD calculations comprise all the elements from the list.

In a more preferred embodiment of the method of the invention, the thrombogenic haemodynamic indexes comprise an endothelial cell activation potential value, and preferably further comprise hypercoagulability and/or blood age values.

In an even more preferred embodiment of the method of the invention, the one or more thrombosis risk factors calculated from the thrombogenic indexes of the patient are determined by comparing each thrombogenic index value with one or more risk threshold values for each thrombogenic index. In a preferred embodiment of the method of the invention, the CFD calculations take as input one or more of the physiological parameters from the list comprising: pulmonary vein pressure, left atrium pressure, doppler ultrasound data and haematocrit levels, preferably wherein the CFD calculations take as input all the elements of said list.

In a preferred embodiment of the method of the invention, the CFD calculations are performed under simulated movement of the left atrium (30) of the patient, preferably wherein the movement is simulated using an Arbitrary Lagrangian-Eulerian method (ALE).

In a more preferred embodiment of the method of the invention, the simulation of the movement comprises the steps of:
h. comparing the 3D model of the left atrium (30) of the patient with a database of 3D models in motion of the left atrium (30) of other patients obtained from dynamic computed tomography images of moving left atriums (30) from other patients;
i. selecting the most similar 3D model of the left atrium (30) from said database;
j. interpolating the movements extracted from the most similar 3D model of the left atrium (30) selected in step b) to the geometry of the left atrium (30) of the patient; and
k. using the interpolated movements to the left atrium (30) of the patient from step c), an arbitrary Lagrangian-Eulerian method (ALE), and the CFD physiological parameters of the patient as input to simulate the movement of the left atrium (30) of the patient.

A second aspect of the invention refers to a computer program comprising instructions which, when the program is executed by a computing means, cause the computing means to carry out the method of any of the previous embodiments of the first aspect.

A third aspect of the invention refers to a data processing apparatus comprising a processing unit and a memory unit, wherein the memory unit comprises instructions which, when executed by the processing unit, configures the data processing apparatus to carry out the method of any of the embodiments of the first aspect.

### Brief description of the drawings

To enable a better understanding of the present disclosure, and to show how the present disclosure may be carried out, reference will now be made, by way of example only, to the accompanying schematic drawings, wherein:
Figure 1 shows a 3D model of a left atrium and an occluder according to one or more embodiments.
Figures 2A and 2B show a 3D model of an occluder positioned inside a 3D model of a left atrial appendage, according to one or more embodiments. More particularly, figures 2A and 2B show an example of an occluder placed inside a LAA according to a first compression value. The chosen positioning of the LAA occluder (10) is subject to modification based on alterations in the landing zone, selection among different computed diameters of the LAA (20), or varying calculated values of the first compression value associated to different positions
Figure 3A shows a simulation of the blood flow inside a left atrium with an occluder positioned proximally inside the left atrial appendage, according to one or more embodiments.
Figure 3B shows a simulation of the blood flow inside a left atrium with an occluder positioned distally inside the left atrial appendage, according to one or more embodiments.
Figure 4 shows three different views (4a, 4b and 4c) of a 3D model of a left atrial appendage occluder with vertical and horizontal sections forming a 3D grid, according to one or more embodiments.
Figure 5 shows a flowchart of a method according to one or more embodiments.
Figure 6 shows a flowchart of a method according to one or more embodiments.

### Description of the invention

### Definitions

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

The term "left atrial appendage (LAA)" refers to a small sac in the muscle wall of the left atrium of the heart. It is known for being a potential site for thrombus formation, especially in patients with atrial fibrillation. The structure and size of the LAA may vary significantly between individuals, thus influencing the selection and planification of occlusion devices.

The term "left atrial appendage occlusion (LAAO)" refers to a medical procedure or the effect thereof, aimed at sealing off the left atrial appendage from the left atrium to prevent the migration of thrombi into the bloodstream, thereby reducing the risk of stroke. In the context of the invention, the occlusion is accomplished by the deployment of an occluder device.

The term "in silico left atrial appendage occlusion (LAAO) surgical planification", in the context of the invention, refers to a preoperative process utilizing computer-based simulations to map out the strategy for LAAO. This involves using computational models and algorithms to analyze patient-specific cardiac anatomy and predict optimal occluder device placement and sizing, thereby facilitating a personalized and precise approach to the occlusion procedure, and is intended to be performed prior to the actual surgical intervention.

The term "compression value", in the context of the invention, refers to a value that represents the extent to which the volume of a body is compacted or condensed. This value may be derived from various assessment methods; for instance, it may be obtained by calculating a ratio indicative of volumetric change to approximate compression, or alternatively, it may be determined through physical simulations that compute the forces and pressures exerted on the body to achieve a specific level of compression.

The term "structural simulation", in the context of the invention, refers to a computational analysis method used to predict how a physical structure behaves under various conditions, such as applied pressures or forces. It involves the use of mathematical models and algorithms to replicate the structural characteristics and response of a device in a virtual environment, taking into consideration a set of boundary conditions and parameters that are critical to the accuracy of the simulation. This simulation may be employed, for example, to calculate a compression value by simulating the physical interactions and deformations that occur during compression.

The term "occluder", in the context of the invention, refers to a left atrial appendage occluder, that is, a device designed to seal off the left atrial appendage (LAA) of the heart, with the aim of reducing the risk of thrombus formation and subsequent stroke in patients, particularly those with atrial fibrillation. The "occluder" is positioned inside the left atrial appendage to prevent the flow of blood to enter inside at least part of the left atrial appendage. Commercial examples of occluders are the Watchman and the Amplatzer Amulet models.

The term "range of acceptance", in the context of the invention, refers to a defined spectrum of values for a given parameter that is established based on criteria related to health, safety, or adherence to medical guidelines and recommendations. This range is determined by an upper and a lower threshold, ensuring that the parameter remains within levels deemed safe and effective for medical practice. The range of acceptance is not static; it may evolve over time as medical standards are reviewed and updated, reflecting the latest research, clinical findings, or regulatory requirements.

The term "thrombosis risk factor", in the context of the invention, refers to any variable or condition that may influence the likelihood of thrombus formation specifically in relation to the placement of an occluder in the left atrial appendage. Within the ambit of LAAO planification, such factors may be derived from, but are not limited to, hemodynamic parameters obtained from computational fluid dynamics simulations and clinical data of a patient. Said thrombosis risk factors may therefore obtain a value depending on the parameter or clinical datum from which are derived, wherein said value may be for instance a weighted value or a binary value.

The term "risk score", in the context of the invention, refers to a calculated value based on a set of thrombosis risk factors. This score provides a metric for evaluating the potential risk of thrombus formation associated with LAAO. A high risk score may prompt the clinician to consider different occluder sizes, types, or positions, to optimize patient outcomes. The methodology for calculating the risk score may involve, for example, aggregating individual risk factors, though the precise algorithm may vary according to specific clinical protocols, medical standards and research data.

The term "landing zone", in the context of the invention, refers to the designated area within the left atrial appendage where the occluder device is initially intended to be placed. The final positioning of the occluder may be adjusted during the LAAO surgical planification to ensure both the stability of the occluder and the minimization of thrombosis risk. The landing zone serves as the preliminary target for the intervention, providing a reference point for potential adjustments during the LAAO procedure. During the LAAO planification the landing zone may be repositioned one or more times depending on a variety of factors, for example on the calculated compression value and/or the risk score.

### Description

Current methodologies for surgical planning of LAAO are limited by their oversimplified approach, often disregarding the intricate haemodynamics and patient-specific anatomical details. Such simplifications result in imprecise assessments of thrombotic risks during a LAAO planification. Additionally, the current computational models, including state-of-the-art computational fluid dynamics (CFD) simulations, lack the sophistication to accurately reflect the personalized blood flow dynamics within the left atrium, and fail to precisely assess the risk of thrombus formation due to the LAAO surgery during the planification stage, which in turn may lead to a stroke.

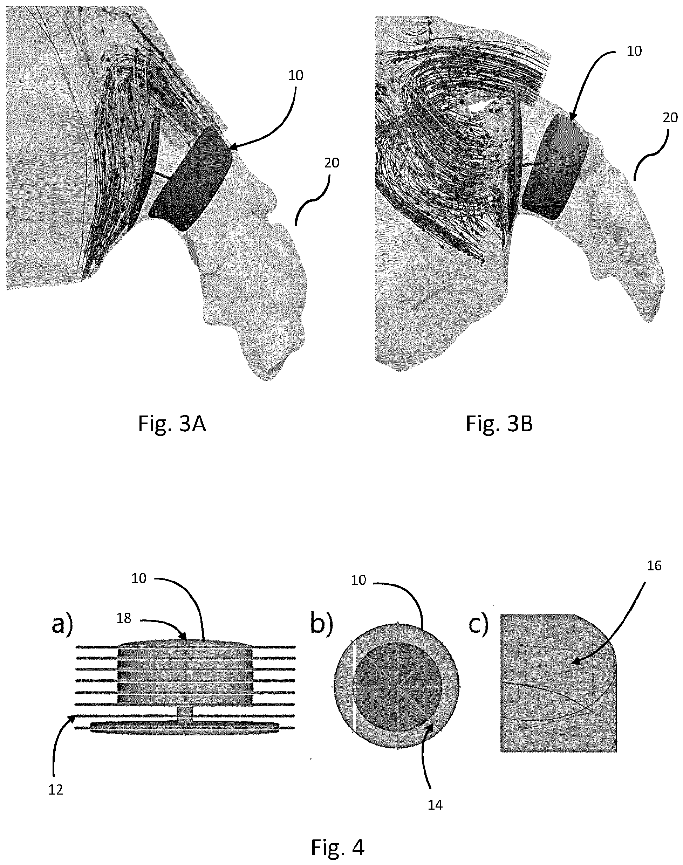

In this invention, as it is backed up by example 1, we provide a method for in silico planning of left atrial appendage occlusion surgical procedures, that solves the above-mentioned problems and avoids undesired outcomes such as device related thrombus (DRT) or peri-device leaks (PDL). As it is shown in example 1, the analysis and computation of the anatomic, haemodynamic and clinical data according to the method of the invention allows the clinician to select a low risk position to place the device. In particular, in example 1 it is shown that the lower risk of device-associated thrombus generation corresponds to the proximal configuration (figure 3A), whereas in the distal configuration (figure 3B) it is more likely that thrombi are generated. For instance, the stagnated streamlines shown in figure 3B that show a recirculation and a lighter color, which indicates reduced velocity, near the wall of the device and are related to a risk factor that may indicate a higher probability of thrombus generation.

This method is personalized to each patient according to its morphology and clinical data, and allows the clinician to interact with the device (LAA occluder) positioning. This is possible thanks to an algorithm that calculates a first compression value in a quick and reliable way, which is an important novelty in the field of LAAO surgical planification. Not only that, a second compression value, mechanically more precise, is also calculated through structural simulations to validate said position, and a computational fluid dynamics simulation is performed to evaluate the relevant parameters that will be used as thrombosis risk factors. Both steps are performed in a patient-personalized way, and, most importantly, taking into consideration a novel combination of relevant morphological and, preferably also clinical, parameters as input, which yields unparalleled results. It is equally important the election of the correct boundary conditions for the simulations carried out in the method of the invention, which may also involve using databases of prior LAAO procedures. Finally, the method uses a combination of said thrombosis risk factors to evaluate the thrombus risk associated to a particular LAA occluder position in a way much more precise than current, even experimental, methods.

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited.

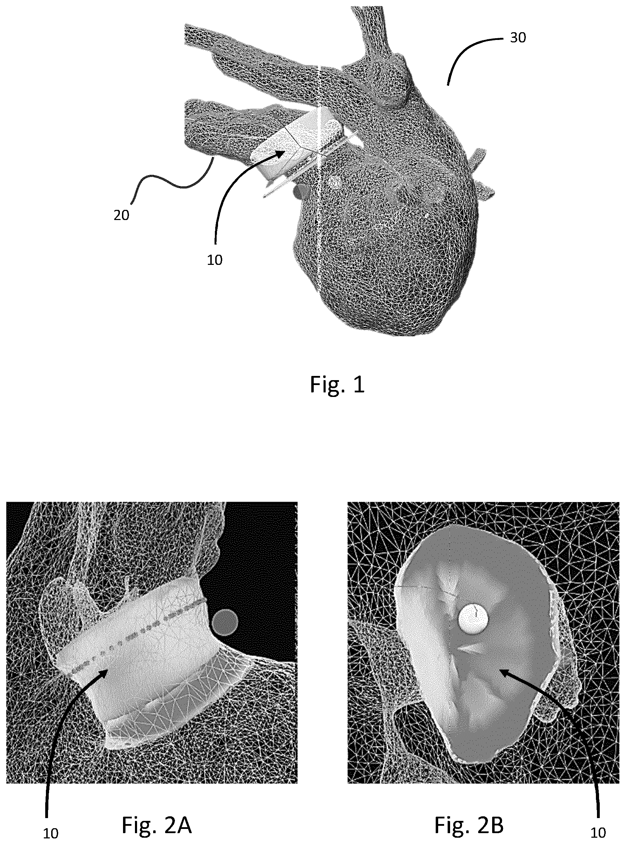

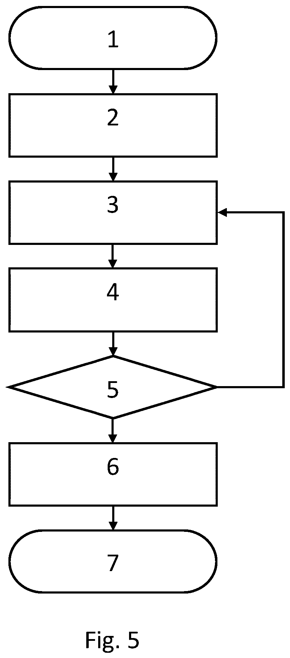

A first embodiment of a first aspect of the invention relates to a computer implemented method (see figure 5 showcasing a flowchart with the numbered steps) for *in silico* surgical planification of a left atrial appendage (20) occlusion procedure. See figures 1, 2 and 3 for 3D representations of a left atrial appendage (20) and an occluder (10), according to one or more embodiments of the invention. The method comprises the following steps (numerated 1,2,3,4,5,6 and 7, according to figure 5, which are equivalent to steps a,b,c,d,e,f,g and h, respectively):

Step (1) (or a) comprises generating a 3D model of the left atrium (30) of a patient departing from medical images of a patient. It is noted that the process of generating a 3D model of the left atrium (30) of a patient comprises acquiring medical images from which the 3D representation may be constructed. In some embodiments, Computed Tomography (CT) or Magnetic Resonance Imaging (MRI) may be utilized, wherein said images are preferably acquired within a period not exceeding one year prior to the generation of the model to ensure the relevance and accuracy of the data. However, it is within the scope of the present invention to employ alternative imaging modalities known to one skilled in the art such as, but not limited to, X-ray angiography, or 3D echocardiography, which may also provide sufficient anatomical detail for the reconstruction of the 3D model. In generating the 3D model of the left atrium (30), the method may involve the use of artificial intelligence, specifically neural network algorithms, to process the acquired images. These neural networks are trained to recognize anatomical structures and are capable of segmenting the left atrium (30) from the surrounding cardiac anatomy with precision. The use of neural networks not only enhances the accuracy of the model but also significantly reduces the time required for its generation, enabling the completion of the model in a matter of minutes. The algorithms may comprise various forms of machine learning algorithms or neural networks such as convolutional neural networks (CNNs), feed forward networks (FFN), or recurrent neural networks (RNNs), among others. Furthermore, the method may alternatively employ manual, semi-automatic, or fully automatic segmentation techniques to aid in the creation of the 3D model. The segmentation process may include thresholding, region-growing, or edge detection techniques, as examples. In some embodiments of the method of the invention, the 3D models may be presented to the user or clinician to any displaying means, and the computing means carring out the method may also provide to the user or the clinician the possibility to modify said 3D models and/or segmentations.

Step (2) (or b) comprises computing one or more inner diameters of the lumen of the left atrial appendage (LAA) (20), wherein said left atrial appendage (20) is comprised in the 3D model of the left atrium (30) of a patient generated in step a). Preferably also comprises computing a centreline along said left atrial appendage (20).

It is noted that the lumen refers to the interior space within the LAA (20) through which blood flows; characteristically, this lumen may present an irregular shape with varying diameters along its length due to the unique anatomical structure of the LAA (20). In some embodiments, the method may compute diameters at multiple cross-sections along a computed centreline of the LAA (20). The computed diameters may include, but are not limited to, the shortest and longest diameters intersecting the centreline at a given point. This computation can be replicated at a plurality of points along the centreline, thus providing a detailed profile of the lumen's dimensions. Furthermore, the diameters may also be calculated as a series of symmetrically spaced radii extending from the centreline out to the inner wall of the LAA (20), capturing the lumen's geometry from multiple perspectives.

Additionally, given the irregularity of the LAA's (20) lumen, the diameters obtained may represent an average diameter, which could be calculated using statistical methods that provide a representative diameter for sections of the lumen. Alternatively, a statistically significant weighted diameter may be computed, accounting for variations in the lumen size due to physiological or pathological conditions. These diameters may be determined using algorithms that are capable of handling non-uniform geometries and are adapted to compute diameters from irregular shapes.

Step (3) (or c) comprises positioning a 3D model of a left atrial appendage occluder (10) at least partially inside the left atrial appendage (20). Said positioning or position is determined according to one or more parameters selected from the list comprising: the one or more diameters calculated in step b), a landing zone position comprised in the 3D model of the left atrial appendage (20), a first compression value of the left atrial appendage occluder (10), and anatomical characteristics extracted from the 3D model of the left atrium (30) of the patient, preferably wherein the anatomical characteristics comprise the shape and position of the ostium and/or the shape and position of the oval fossa.

It is noted that the determination of the optimal position for the LAA occluder (10) is based on a multiplicity of parameters, which may include the one or more inner diameters computed in step (b), the location of a landing zone within the 3D model of the LAA (20), a first compression value of the LAA occluder (10), and various anatomical characteristics discernible from the 3D model of the left atrium (30). It is further noted that the landing zone may be characterized as a specific surface or point within the LAA (20), or, in some other embodiments, as a suitable volume within the LAA (20). Said landing zone may serve as starting point, and may be identified and selected through manual intervention by an user or a clinician or automatically, for instance by referencing a pre-established database of landing zones utilized in previous LAA occlusion (LAAO) procedures. It is further noted that the first compression value may be a volumetric approximation, in the sense of a relationship between volumes or sub-volumes, or between points comprised in said volumes, and not a mechanical simulation. For example, the first compression value may be determined by the interaction between the occluder (10) and the LAA (20) in terms of volumes, sub-volumes, or specific points therein. Importantly, this first compression value is not indicative of a mechanical compression simulation but rather a geometric relationship that influences the initial placement of the occluder (10). Figure 2A and 2B show an example of an occluder placed inside a LAA according to a first compression value. The chosen positioning of the LAA occluder (10) is subject to modification based on alterations in the landing zone, selection among different computed diameters of the LAA (20), or varying calculated values of the first compression value associated to different positions. In certain embodiments, a series of first compression values might be computed to evaluate different potential positions of the occluder (10). The ultimate selection of a particular compression value may be guided by criteria pertaining to the adequacy, safety, or performance projected by these values. Furthermore, in some embodiments the computing means may offer a range of alternative positions for the LAA occluder (10) from which the clinician may choose. These proposed positions may be based on the ratio of diameters between the LAA (20) and the LAA occluder (10). The diameter of the LAA (20) considered in this context may be an average, minimum, maximum, or another statistically representative measure. Additionally, for each proposed position, there may be an associated distal and proximal recommendation relative to the ostium of the LAA (20), with 'proximal' referring to a position nearer to the ostium of the LAA (20) and 'distal' referring to a location further from it, as delineated in figures 3A and 3B, respectively. This dual positioning suggestion accommodates variations in the anatomical structure of the LAA (20) and aims to ensure the most effective engagement of the LAA occluder (10).

Step (4) (or d) comprises performing a structural simulation to calculate a second compression value of the left atrial appendage occluder (10) from its interaction with the inner walls of the left atrial appendage (20). Preferably, said structural simulation is made through a finite element method (FEM), more preferably it provides values of pressure and exerted forces.

It is noted that a skilled person may perform the structural simulations to ascertain the mechanical behavior of the occluder (10) through other methods, such as finite difference method (FDM), finite volume method (FVM), spectral methods, boundary element method (BEM) and/or Meshless methods. It is noted, that when calculating the interaction of the LAA occluder (10) with the inner walls of the left atrial appendage (20) said inner walls may be considered as non-deformable as boundary condition. The simulation may take into account material properties of the occluder (10), which may comprise biocompatible metals such as, but not limited to, titanium, nitinol, stainless steel, or cobalt-chromium alloys, or polymers including, but not limited to, silicone, polyurethane, or PTFE. The properties of these materials, such as elasticity, plasticity, and viscoelasticity, may be modelled to predict the occluder's (10) deformation under physiological conditions. Moreover, the structural simulation may consider the inner walls of the left atrial appendage (20) as either non-deformable or deformable with varying degrees of rigidity. This takes into account patient-specific variations, which may alter the interaction between the occluder (10) and the atrial tissue. In some embodiments, the inner walls may be assigned material properties similar to cardiac tissue to simulate realistic interactions. According to some embodiments, the structural simulation may provide values of pressure and exerted forces that are consequential for assessing the stability and sealing capability of the occluder (10). Such information may be critical for determining the effectiveness of the occlusion and the likelihood of migration or leakage post-implantation. In other embodiments of the invention, the simulation may also consider dynamic conditions such as the pulsatile nature of blood flow or cardiac cycle-induced motion of the left atrial appendage (20) walls, thus ensuring a comprehensive assessment under near-physiological conditions.

In step (5) (or e), if the previously calculated second compression value in step d) is within a range of acceptance, the method is followed by proceeding with step f). However, if the second compression value calculated in step d) has a value outside said range of acceptance, then the method is continued by going back to step c) and performing step c) again with one or more parameters modified, wherein said parameters are preferably the landing zone, the first compression value and/or the one or more diameters.

It is noted that said range of acceptance for the second compression value may be derived from a compilation of clinical data, expert consensus, or defined by the specifications provided by the manufacturers of the left atrial appendage occluder (10). A skilled person may recognize these ranges as typical for ensuring the efficacy and safety of LAAO procedures.It is further noted that when repeating step c) the parameters may be modified automatically, and, in some other embodiments, the parameters may be modified manually. These parameters may include, but are not limited to, the landing zone, the first compression value, and the one or more diameters of the left atrial appendage (20). The modification of these parameters, such as the landing zone and the one or more diameters of the LAA (20) may lead to a new set of first compression values, hence affecting the positioning of the occluder (10) and subsequently, the second compression value obtained in step d). Similarly, choosing a different first compression value would yield a different positioning of the occluder (10), since each first compression value may be associated with a particular positioning of the occluder (10). In embodiments where the system facilitates automatic adjustments, algorithms may be employed to optimize parameters based on historical data, 14 simulations outcomes, or a database containing an array of successful LAAO procedure parameters. The system may utilize artificial intelligence to suggest parameter modifications that maximize the likelihood of falling within the acceptable range upon reevaluation in step c) and/or e). Manual adjustment, on the other hand, allows the clinician to apply nuanced expertise and make informed decisions on parameter changes. This approach can integrate patient-specific considerations that may not be fully captured by automated systems. Moreover, a combination of modified parameters may be employed when repeating step c), offering a multifaceted approach to optimization. Modifications may follow a deterministic, random, or similarity-based pattern, referencing a comprehensive database of LAAO procedures and simulations to inform the selection process.

Step (6) (or f) comprises calculating a set of thrombosis risk factors using parameters derived from computational fluid dynamics (CFD) calculations and using medical parameters of the patient. It is noted that the thrombosis risk factors may be individual numerical values that correlate with specific parameters from the CFD calculations. In certain embodiments, multiple CFD-derived parameters may be combined to constitute a single risk factor. The method of combining these parameters may be varied and may include weighted averaging, statistical modeling, or any correlation deemed appropriate by those skilled in the art. Likewise, the risk factors emerging from the patient's medical parameters may also be constituted by single values or an aggregate of multiple values, which are collectively considered to provide a comprehensive risk assessment. In some embodiments, the grouping and/or the determination of these medical parameters as risk factors are carried out following medical guidelines, protocols, or standard practices established by national or international medical authorities, such as the European Society of Cardiology (ESC) or the American Heart Association (AHA). In other embodiments of the invention, the CFD calculations may also consider dynamic conditions such as the pulsatile nature of blood flow or cardiac cycle-induced motion of the left atrial appendage (20) walls, thus ensuring a comprehensive assessment under near-physiological conditions.

Step (7) (or g) comprises computing a risk score of developing thrombosis related to the left atrial appendage (20) occlusion positioning from step c), wherein said risk score is at least based on the thrombosis risk factors calculated in step f). It is noted that said risk score may be computed as a sum of risk factors, as a weighted sum of risk factors or following any mathematical relationship between the risk factors, wherein said risk factors may also interact synergistically to increase or multiply said risk factor to a value higher than their separate contribution, or, alternatively, they may interact synergistically to decrease or divide said risk factor to a value lower than their separate contribution. It is further noted that the risk score may be provided to the user or the clinician through displaying means, and it may be associated to a recommendation regarding the placement of the LAA occluder (10) or to a recommendation to repeat the method by changing some parameters.

It is also noted that the disclosed method for in silico planning of left atrial appendage occlusion (LAAO) surgical procedures may be implemented through various computational frameworks. In some embodiments, the method may be integrated into a web-based platform, allowing for remote and centralized access, thus facilitating collaborative and multidisciplinary planning. Additionally, the method may be deployed on a cloud system, offering scalable computing resources and storage capabilities, ensuring the method's accessibility and efficiency irrespective of the user's local hardware limitations. Alternatively, in some other embodiments the method may be encapsulated within a standalone software application, designed for execution on local computing systems, providing users with the capability to perform the planning process without the necessity of an internet connection. Such standalone applications may be tailored for compatibility with various operating systems and hardware specifications, ensuring a wide range of usability. It is further noted that the method is distinctly extracorporeal, designed to be executed outside of the human body and is not intended for intraoperative use. Therefore, the method is conceptualized for utilization during the preoperative phase of the surgical planning process. By conducting the method in silico, a detailed and precise surgical plan can be formulated, taking into account patient-specific anatomical and physiological data. This prior planning aims to enhance the surgical outcomes by enabling the surgical team to anticipate potential challenges and to tailor the intervention to the individual characteristics of each patient's left atrial appendage (20).

It is further noted that in some embodiments the method of the invention may be integrated into a web-based platform, a cloud system, a standalone software, or any other appropriate computer means. It is also noted that the method of the invention is to be carried out *in silico,* that is, it is extracorporeal, and therefore it is not directed to being carried out during a surgical procedure. Instead, it is directed to be carried out previously, during the planification stage of a LAAO surgical procedure.

Advantageously, the claimed computer-implemented method for in silico surgical planification of a left atrial appendage (20) occlusion (LAAO) presents several significant benefits. For instance, this method provides a quick and precise calculation of a first compression value, which allows to interactively visualize and change the position of the occluder (10), or obtaining, within minutes or seconds, a positioning suggestion based on this fast and reliable first compression value. Said first compression value is fundamental in order to select a position that does not damage the LAA nor there is the risk of it being displaced or failing to seal the LAA. Therefore, having a refined method that uses patient parameters, such as the ostium and/or the oval fossa, and geometrical considerations to quickly provide safe positioning suggestions is very advantageous. This ensures that the occluder is appropriately placed within the LAA (20), achieving a first compression value that supports both the stability and the effectiveness of the occluder. Furthermore, by calculating a set of thrombosis risk factors using computational fluid dynamics calculations alongside medical parameters of the patient, the method provides a sophisticated prediction of thrombosis risk, which is a growing concern in LAAO procedures. The subsequent computation of a risk score for developing thrombosis from the occluder's (10) positioning offers a quantifiable assessment of the procedure's safety, aiding clinicians in making informed decisions. In summary, the claimed method provides a comprehensive, tailored surgical planning tool that maximizes the efficacy and safety of LAAO procedures. The integration of precise anatomical modeling with dynamic occluder positioning and thrombosis risk assessment ensures a high-quality outcome that is both patient-specific and reliable, thereby reducing overall procedural risk and promoting successful surgical interventions.

According to another embodiment of the method of the invention, if the risk score computed in step g) is above a risk threshold value, the next step is modifying the landing zone position, the first compression value and/or the one or more diameters selected from the one or more diameters calculated in step b), and performing step c) again.

It is noted that the method allows for an adaptable response when a computed risk score surpasses a designated risk threshold value, signifying the need for modification of key parameters. Said risk threshold value may be based on recommendations from medical authorities such as the ESC or the AHA, and it therefore may vary over time, or it may be adjusted by a skilled person following research or hospital practices. In some embodiments, the alteration of the landing zone position, the first compression value, and/or the selected diameters can be manually conducted, offering clinicians the flexibility to apply their judgment based on experience and specific patient anatomy. In some other embodiments adjustments to these parameters may be performed automatically by the system, employing algorithms that adaptively seek an optimal configuration. This can enhance the precision of the surgical plan by utilizing systematic computational methods to determine the most favorable adjustments. In some other embodiments the modifications may be executed randomly to thoroughly investigate various potential outcomes, or in accordance with a comprehensive database of historical successful placements, which provides empirical evidence to inform the modification process. The parameters might be individually or collectively adjusted, thus enabling a multifaceted approach to the optimization that addresses the complex interplay between different factors affecting the occlusion outcome.

Advantageously, this step allows the clinician or user to repeat some steps of the method by modifying key parameters until a safe position of the LAA occluder is found.

According to another embodiment of the method of the invention, if the risk score computed in step g) is above a risk threshold value, the next step is displacing the 3D model of the left atrial appendage occluder (10) to a different position according to a database of CFD simulations performed on LAAO from other patients and repeating step d), preferably wherein the different position minimizes the risk score according to a matching algorithm that extracts said different position from matching the occluder (10) with another similar left atrial appendage occluder (10) from a simulation database.

It is further noted that the method preferably utilizes a matching algorithm, which aids in minimizing the risk score by selecting an alternative position for the occluder (10). The algorithm functions by comparing the current patient's LAA anatomy and occluder (10) position with archived simulation data and parameters of similar anatomical structures and occluder positions. Through this comparison, the algorithm can extract a position that has previously resulted in a reduced risk score in the database, thus potentially offering a safer and more efficacious occlusion strategy for the current patient.

Advantageously, this approach leverages historical data and advanced matching techniques to enhance the decision-making process, thereby reducing the trial-and-error aspect of positioning the occluder (10). By aligning the occluder (10) position with data-backed simulations, the method significantly increases the likelihood of achieving an optimal occlusion outcome with a lower risk of thrombosis. This informed method of displacement not only personalizes the procedure to the patient's specific anatomy but also incorporates collective insights from past successful interventions, potentially leading to improved procedural success rates and patient outcomes.

According to another embodiment of the method of the invention, the medical images of the patient are generated from one or more imaging techniques from the list comprising: ultrasound (US) imaging, Transthoracic Echocardiography (TTE), Computed Tomography (CT) imaging, Magnetic Resonance Imaging (MRI) imaging, 3D Rotational Angiography (3DRA) imaging, mitral valve pulsatile ultrasound imaging, and pulmonary veins pulsatile ultrasound imaging; preferably wherein the 3D model of the left atrium (30) of the patient is generated using input images generated using mitral valve pulsatile US imaging and either one of MRI, 3DRA or CT imaging; more preferably wherein he 3D model of the left atrium (30) of the patient is generated using input images generated using mitral valve pulsatile US, pulmonary valve pulsatile US, and either one of MRI, 3DRA or CT imaging.

Advantageously, the preferred combination of these imaging techniques balances the high temporal resolution of pulsatile US imaging with the spatial resolution and anatomical detail provided by MRI, 3DRA, or CT imaging, leading to a comprehensive representation of the left atrium (30). Also, using input images from both mitral valve pulsatile US and pulmonary valve pulsatile US to generate the 3D model of the left atrium (30) further enriches the 3D model with dynamic information about the blood motion, which is crucial for accurate device positioning and risk assessment. On the other hand, the use of multiple imaging techniques in generating the 3D model of the left atrium (30) significantly enhances the accuracy and reliability of the model. This multi-modal imaging strategy allows for a detailed understanding of the patient's unique cardiac anatomy and dynamics, which is instrumental in creating a tailored surgical plan. Moreover, the utilization of pulsatile ultrasound imaging provides real-time data on blood and heart movement, which can be critical for the precise placement of the occluder (10) and for assessing potential risks such as device interference with valvular function. This methodological flexibility in image sourcing and model creation inherently improves the fidelity of the surgical planning process, thereby increasing the probability of a successful occlusion procedure and patient outcome.

According to another embodiment of the method of the invention, the first compression value is based on a volumetric relationship between the volume defined by the unmodified 3D model of the occluder (10) and the volume defined by the 3D model of the occluder (10) when its volume is modified to fit to the dimensions of the internal walls of the 3D model of the left atrium (30) of the patient, preferably wherein the 3D model of the occluder (10) is virtually divided according to one or more sections (12, 14) perpendicular to, or departing from, a centreline (18) of the 3D model of the occluder (10), wherein said sections (12, 14) define two or more sub-volumes (16) and each section comprises one or more points along each of said sections and wherein the first compression value is approximated as a relationship between the position of said points when the occluder (10) is non-compressed and the position of said points when the occluder (10) is compressed to fit to the dimensions of the internal walls of the 3D model of the left atrium (30) of the patient.

It is noted that a the volumetric relationship may refer to the comparative analysis between two volumes: the inherent volume of an unmodified 3D model of the occluder (10) and the altered volume when the occluder (10) is adapted to align with the spatial constraints of the left atrial appendage (20). This relationship quantifies how much the occluder (10) must compress or expand to fit within the unique anatomical features of the left atrium (30) of the patient. It is further noted that the unmodified 3D model of the occluder (10) denotes the occluder's (10) geometry as initially designed or manufactured, prior to any alterations for fitting within the left atrial appendage (20). Conversely, the modified volume of the occluder (10) corresponds to its altered state post-adaptation, where its dimensions are conformed to the internal contours of the left atrium (30). Regarding the sectioning, it is noted that the 3D model of the occluder (10) may be divided into discrete sections (12, 14), which could be perpendicular or at varying angles to a centerline (18) of the occluder (10). This sectioning may take various forms, including, but not limited to, radial, axial, or arbitrary sections, accommodating to any geometrical possibility known to the skilled person. This flexibility is instrumental in simplifying the complexities associated with the occluder's (10) deformation analysis, as it facilitates the breakdown of the structure into manageable segments that can be individually assessed and adjusted. It is further noted that sub-volumes (16) are the individual volumes delineated by the sections (12, 14) of the occluder (10). These sub-volumes (16) represent partitions of the overall volume of the occluder (10) and are crucial for computing how each section (12, 14) contributes to the total compression value. It is also noted that the "points" located along each section (12, 14) serve as reference coordinates for tracking the deformation of the occluder (10). These points, before and after compression or modification, establish a relationship that provides the degree of displacement required for the occluder (10) to achieve optimal conformity with the inner walls of the left atrium (30). The positional shift of these points, from an uncompressed to a compressed state, defines the first compression value. This value is pivotal in determining the suitability of the occluder (10) for effective occlusion while maintaining its structural integrity. Advantageously, the adoption of a volumetric assessment for determining the first compression value enhances the precision of the occluder's (10) sizing and fit, and it greatly boosts the speed of the calculations compared with a mechanical simulation. The segmentation into sections (12, 14) provides a detailed approach to adapting the occluder (10), allowing for adjustments that account for the complex geometry of the left atrial appendage (20). This granular approach to 19urther19g the occluder (10) ensures that the device conforms effectively to the patient-specific anatomy, which can mitigate complications such as device embolization or peri-device leaks, and thus, improve patient outcomes. The use of sub-volumes (16) and sections (12, 14) yields a computational model that is accurate in its predictive capacity, fast and versatile in application, thereby enhancing the operational success of the occlusion method.

According to another embodiment of the method of the invention, the position of the LAA occluder determined in step c) is further determined according to clinical data of the patient, preferably wherein the clinical data of the patient comprises one or more of the elements from the list comprising: CHADS2-VASc, HAS-Bleed and ultrasound data; more preferably the clinical data further comprises one or more elements from the list comprising: left atrium pressure, haemoglobin levels, BNP, C-Protein levels, Troponine levels, eGFR, ECG data, and creatinine levels; even more preferably wherein the clinical data of the patient comprises all the elements of both lists.

It noted that the patient's clinical data, such as the CHADS2-VASc score, are not static and may be subject to change over time as per the guidelines of authoritative bodies such as the European Society of Cardiology (ESC) and the American Heart Association (AHA). It is also noted that the incorporation of such clinical data may be realized through computational methods or algorithms that factor these variables into the occluder (10) placement process.

Advantageously, the employment of an expansive array of clinical data including, but not limited to, CHADS2-VASc, HAS-Bleed, ultrasound data and preferably other physiological biomarkers, facilitates a holistically informed method of LAA occluder (10) placement. These elements provide a multifaceted view of the patient's health status, which can be critical for the accurate positioning of the occluder (10). This method, by leveraging comprehensive patient-specific data, aims to maximize the therapeutic benefits of the LAA occlusion procedure, potentially reducing the incidence of adverse events and optimizing long-term patient outcomes.

According to another embodiment of the method of the invention, the position of the LAA occluder determined in step c) is further determined according to at least one or more elements from the list comprising: a landing zone position comprised in the 3D model of the left atrial appendage (20), a database of left atrial appendage occluders (10) positioned in the left atrial appendage (20) of other patients, mechanic and volumetric characteristics of a catheter, wherein said catheter is associated to the positioning of the left atrial appendage occluder (10), and ultrasound images; preferably wherein the position of the LAA occluder is determined according to all the elements of said list.

It is further noted that a repository or database containing records of LAA occluders (10) positioned in the LAAs (20) of a plurality of other patients may serve as a reference for determining the optimal position of the occluder (10). This database may include data points from previous successful implantations, thus providing an empirical basis to support positioning decisions. Said database and a matching algorithm may be used to determine the optimal configuration of a left atrial appendage occluder (LAAO) for a patient based on their individual characteristics and the characteristics of LAAOs that have been previously implanted in other patients. The algorithm works by first extracting morphological features and clinical characteristics from the patient, such as haematocrit levels and creatinine levels. These features are then compared to a database of LAAOs that have been previously implanted in other patients, using a similarity metric based on the Euclidean distance. The Euclidean distance is a measure of the distance between two points in space, and in this case, it is used to measure the difference between the patient's characteristics and the characteristics of the LAAOs in the database. In some embodiments, the algorithm may calculate the sum of the absolute differences between the patient's features and the features of each LAAO in the database, and select the LAAO with the smallest sum as the optimal configuration for the patient. It is further noted that the mechanic and volumetric properties of the catheter used in the positioning process are also considered as part of the occluder placement strategy. These properties may affect the manoeuvrability and the ultimate positioning of the occluder (10), and therefore, their influence on the procedure may be accounted for in the method.

Advantageously, incorporating a diverse set of parameters in the determination of the position of the occluder (10) that includes the specific landing zone, a database of prior occluder placements, catheter characteristics, and live ultrasound imaging enables a detailed and highly informed approach to occluder (10) positioning. Utilizing a composite of these elements may enhance the customizability of the procedure to individual patient anatomy and conditions, leading to improved patient-specific outcomes and reduced risk of complications associated with suboptimal occluder positioning.

According to another embodiment of the method of the invention, the medical parameters of the patient comprise CHADS2-VASc and/or HAS-Bleed.

It is noted that CHADS2-VASc stands for Congestive heart failure, Hypertension, Age ≥75 years, Diabetes mellitus, Stroke/transient ischemic attack, Vascular disease, Age 65-74 years, Sex category. Each of these factors is assigned a score, and the total score is used to estimate the risk of stroke in patients with atrial fibrillation. On the ther hand, HAS-BLED stands for Hypertension, Abnormal renal/liver function, Stroke, Bleeding history or predisposition, Labile international normalized ratio, Elderly (>65), Drugs/alcohol concomitantly. Each of these factors is assigned a score, and the total score is used to estimate the risk of bleeding in patients taking anticoagulant medication.

It is further noted that the inclusion of these scores allows for a tailored approach to the placement of the left atrial appendage occluder (10). This individualized strategy ensures that the risk of thromboembolic events and bleeding complications is minimized by considering the patient's unique risk profile as determined by these scores.

Advantageously, incorporating CHADS2-VASc and HAS-Bleed scores into the medical parameters considered by the method provides a means to align the therapeutic intervention with the patient's clinical needs. This approach may reduce the likelihood of post-procedural complications, enhance the efficacy of the occluder (10) placement, and potentially contribute to a more favorable prognosis for the patient.

According to another embodiment of the method of the invention, the thrombosis risk factors derived from CFD calculations comprise one or more of the elements from the list comprising: the average blood velocity in the pulmonary ridge area, the presence and average blood velocity of eddies, and/or stagnated flow in the pulmonary ridge area and device surface, the particle attachment quantity, and thrombogenic haemodynamic indexes; preferably wherein the thrombosis risk factors derived from CFD calculations comprise all the elements from the list.

Advantageously, by assessing a comprehensive set of thrombosis risk factors derived from CFD calculations, the method may proactively mitigate the risk of thrombus formation. The thorough analysis of these factors allows for the optimization of the occluder's (10) design and positioning, thereby enhancing the safety and effectiveness of the procedure. Including these thrombosis risk factors ensures that the intervention is not only tailored to the patient's anatomical requirements but also to the dynamic conditions within the LAA, which may vary significantly between individuals. This holistic approach may improve patient outcomes by reducing the incidence of procedure-related thrombotic events.

According to another embodiment of the method of the invention, the thrombogenic haemodynamic indexes comprise an endothelial cell activation potential value, and preferably further comprise hypercoagulability and/or blood age values.

Advantageously, the incorporation of endothelial cell activation potential, along with hypercoagulability and blood age values, into the thrombogenic hemodynamic indexes enables a more nuanced and comprehensive assessment of thrombosis risk. This facilitates the optimization of the LAA occluder (10) positioning and design, potentially leading to more individualized and effective thrombosis prevention strategies.

According to another embodiment of the method of the invention, the one or more thrombosis risk factors calculated from the thrombogenic indexes of the patient are determined by comparing each thrombogenic index value with one or more risk threshold values for each thrombogenic index.

It is noted that these risk threshold values may as critical points of reference that can indicate an elevated risk of thrombus formation when exceeded by the measured index values. In some embodiments, there may be different risk degrees or thresholds, instead of only one threshold value, that define different risk levels, therefore contributing gradually or differently to the value of each risk factor.

The determination of these risk threshold values for each thrombogenic index may be established by medical authorities, such as the European Society of Cardiology (ESC) or the American Heart Association (AHA). Alternatively, or in addition, threshold values may be defined by hospital practices based on clinical studies or patient outcomes. These thresholds may evolve over time as new research provides deeper insights into the factors influencing thrombosis. Moreover, variation may exist across different regions due to differing medical protocols, population genetics, and prevalent local practices.

Advantageously, the approach in determining the positioning and deployment of the LAA occluder (10) based on these comparative analyses may lead to a more standardized and potentially safer patient experience.

According to another embodiment of the method of the invention, the CFD calculations take as input one or more of the physiological parameters from the list comprising: pulmonary vein pressure, left atrium pressure, doppler ultrasound data and haematocrit levels, preferably wherein the CFD calculations take as input all the elements of said list.

Advantageously, by accommodating a broad spectrum of physiological parameters, the method can yield a more detailed simulation of the patient's cardiac environment. This comprehensive approach may facilitate the identification of optimal occluder placement and configuration, enhancing the predictability of procedural outcomes. Including such a variety of inputs also enables personalization of the simulation to the patient's unique physiological context, potentially increasing the precision of the occluder fitting and reducing the likelihood of complications such as thrombosis or device migration.

According to another embodiment of the method of the invention, the CFD calculations are performed under simulated movement of the left atrium (30) of the patient, preferably wherein the movement is simulated using an Arbitrary Lagrangian-Eulerian method (ALE).

Advantageously, incorporating simulated movement of the left atrium (30) via an ALE method into the CFD calculations allows for a sophisticated analysis of the mechanical stresses and fluid-structure interactions that the occluder (10) will experience in vivo. This can significantly improve the predictive power of the simulation, potentially reducing the risk of complications by ensuring that the device's behaviour under physiological conditions is thoroughly understood and accounted for. This results in a more tailored and potentially safer approach to device implantation for each individual patient.

According to a preferred embodiment of the method of the invention, the simulation of the movement comprises the steps of:
a) comparing the 3D model of the left atrium (30) of the patient with a database of 3D models in motion of the left atrium (30) of other patients obtained from dynamic computed tomography images of moving left atriums (30) from other patients;
b) selecting the most similar 3D model of the left atrium (30) from said database;
c) interpolating the movements extracted from the most similar 3D model of the left atrium (30) selected in step b) to the geometry of the left atrium (30) of the patient; and
d) using the interpolated movements to the left atrium (30) of the patient from step c) , an arbitrary Lagrangian-Eulerian method (ALE), and the CFD physiological parameters of the patient as input to simulate the movement of the left atrium (30) of the patient.

It is noted that other alternatives to dynamic computed tomography images for representing the moving left atrium (30) may include, but are not limited to, magnetic resonance imaging (MRI) sequences, echocardiograms, particularly 3D echocardiography, and cardiac catheterization data.

It is also noted that in some embodiments the comparison of the 3D model of the patient's left atrium (30) with the models within the database may be performed using various computational techniques known to the skilled person such as geometric morphometrics, which analyze shapes, or registration algorithms that seek to overlay two models to quantify their similarity. In some embodiments, parameters such as size, shape, motion pattern, and/or wall thickness of the left atrium (30) may also be considered in this comparison. It is further noted that by the term "most similar" when selecting the most similar 3D model of the left atrium (30) may refer to the model with the highest degree of congruence across a range of metrics, including geometric dimensions, physiological motion patterns, and/or tissue characteristics. This assessment may be qualitative or quantitative, potentially involving machine learning algorithms trained to recognize patterns that align with the patient's data. Additionally, in some embodiments the interpolation may involve techniques such as spline interpolation or shape-based averaging. Alternatively to interpolation, some embodiments may comprise statistical shape modelling or the use of deep learning models that can predict the patient-specific motion based on learned patterns from a wide range of previously analyzed movements. It is further noted that the simulation of the movement with the inputs mentioned may utilize physics-based models to replicate the mechanical behavior of the left atrium (30) under cardiac load conditions. In some embodiments other simulation techniques may be employed such as finite element analysis or fluid-structure interaction models, which can also account for complex interactions between the blood flow and the atrial wall motion.

Advantageously, this process enables the creation of a patient-specific simulation that accounts for the unique movements of the left atrium (30), thereby providing a more accurate prediction of the left atrial appendage occluder's (10) behavior within the dynamic cardiac environment. This could significantly aid in optimizing the fit and placement of the occluder (10), potentially reducing the risk of displacement or other complications, resulting in a more effective treatment tailored to the individual patient's anatomy and cardiac dynamics.

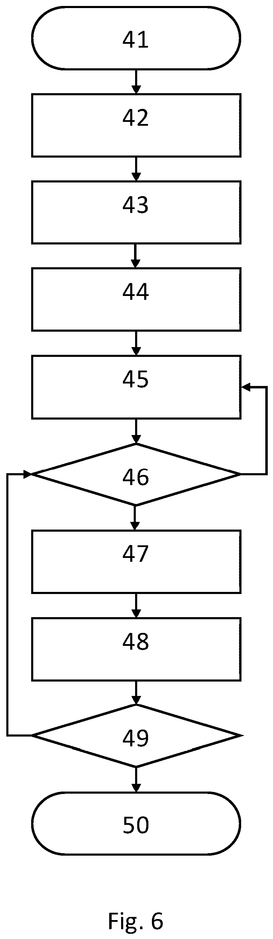

Figure 6 depicts an exemplary embodiment of the invention (wherein numbers 41, 42, 43, 44, 45, 46, 47, 48, 49, and 50 correspond to the letters a, b, c, d, e, f, g, h, i, j, respectively). Figure 6 shows a computer-implemented method for in silico surgical planning of a left atrial appendage (LAA) occlusion (LAAO) comprising the steps of:
a. uploading data comprising medical images and clinical data of a patient;
b. generating a three-dimensional (3D) model of the left atrium (30) of the patient from the uploaded medical images;
c. computing various anatomical features of the left atrium (30) and the left atrial appendage (LAA) (20) within the 3D model generated in step b);
d. executing a compression approximation approach to estimate a first compression value of a left atrial appendage occluder (10);
e. providing at least one suggested position for the left atrial appendage occluder (10) based on a matching algorithm that inputs diameters from step c), a landing zone, anatomical characteristics from the 3D model, and the first compression value from step d);
f. performing a structural simulation to determine a second compression value of the left atrial appendage occluder (10); if the second compression value is not within acceptable ranges, modifying one or more parameters and repeating step e);
g. conducting flow simulation using computational fluid dynamics (CFD) and arbitrary Lagrangian-Eulerian (ALE) methodologies, utilizing clinical data, ultrasound imaging, and simulated movement of the left atrium (30) as inputs;
h. calculating a device-related thrombosis (DRT) risk score based on parameters derived from the simulations conducted in step g);
i. if the DRT risk score is above a risk threshold value, finding an optimized position of the left atrial appendage occluder (10) by using a simulation database and an additional matching algorithm, then returning to step f) with the newly suggested position. If the DRT risk score is equal or below a risk threshold value, performing step j.
j. finishing the algorithm, preferably displaying the DRT risk score before finishing the algorithm.

A second aspect of the invention refers to a computer program comprising instructions which, when the program is executed by a computing means, cause the computing means to carry out the method of any of the previous claims.

It is noted that said program may be configured to process 3D models, assess volumetric relationships, integrate patient-specific clinical data, and perform complex computational fluid dynamics (CFD) calculations. Also, the instructions within the program may be structured to interact with databases of anatomical models, execute algorithms for comparing and interpolating physiological movements, and apply various methods of simulation such as the Arbitrary Lagrangian-Eulerian (ALE) method. Additionally, the program may include modules for analyzing thrombosis risk factors and integrating a multitude of patient-specific medical parameters. Alternatives for the computing means on which the program runs may include standalone workstations, integrated systems within medical devices, cloud-based computing environments, or distributed computing platforms, allowing for flexibility in deployment.

Advantageously, the execution of such a computer program facilitates a highly individualized approach to the treatment of conditions such as atrial fibrillation, enabling clinicians to make informed decisions based on a confluence of simulation and real-world data, potentially improving patient outcomes through the customized design of medical interventions.

A third aspect of the invention refers to a data processing apparatus comprising a processing unit and a memory unit, wherein the memory unit comprises instructions which, when executed by the processing unit, configures the data processing apparatus to carry out the method of the invention.

It is noted that the data processing apparatus may comprise various forms of hardware such as a central processing unit (CPU), a graphics processing unit (GPU), or specialized processors like a digital signal processor (DSP) or an application-specific integrated circuit (ASIC). It may further be adapted to perform high-speed computations required for three-dimensional modelling and Computational Fluid Dynamics (CFD) simulations. The memory unit may encompass non-volatile forms of storage such as read-only memory (ROM), flash memory, or solid-state drives (SSDs) which retain the necessary instructions. It may also include volatile memory such as random-access memory (RAM) to facilitate quick access and execution of instructions by the processing unit. The data processing apparatus may comprise additional components such as input/output interfaces for interacting with peripheral devices, network connectivity modules to access patient data and databases, and user interface elements to display results and facilitate clinician interaction. Advantageously, this configuration allows for a self-contained system capable of delivering sophisticated analyses pertinent to the positioning of LAA occluders, informed by patient-specific data and advanced modelling techniques. This may optimize procedural outcomes and support personalized medical treatment strategies.

A fourth aspect of the disclosure refers to a computer implemented method for in silico determination of a risk of developing thrombosis related to a left atrial appendage occlusion. This fourth aspect refers to a method that is independent of the first aspect of the invention, as it is a method relating the determination of a risk of developing thrombosis, instead of to a planification method. The method comprises the steps of:
a. departing from a 3D model of the left atrium of a patient comprising a 3D model of a left atrial appendage occluder, performing computational fluid dynamics (CFD) calculations on the blood flow through said 3D model of the left atrium taking patient parameters as input, wherein said input patient parameters comprise at least haematocrit levels and/or specific blood pressure,
b. obtaining a set of parameters from the CFD calculations, wherein said set of parameters comprises one or more parameters selected from the list comprising: the average blood velocity in the pulmonary ridge area, the average blood velocity of eddies, streamlines and/or stagnated flow, the particle attachment quantity, and thrombogenic indexes, wherein the thrombogenic indexes comprise at least one or more of the following parameters: hypercoagulability, blood age, and/or endothelial cell activation potential;
c. computing a risk score of developing thrombosis related to a left atrial appendage occlusion in a patient, at least based on each parameter extracted from the computational fluid dynamics (CFD) calculations in step b, and clinical data of the patient, wherein said clinical data of the patient comprises at least CHADS2-VASc and/or HAS-Bleed;
d. comparing the risk score with at least one or more predefined values that separate different degrees of risk of developing thrombosis;
e. determining the risk of developing thrombosis related to a left atrial appendage occlusion according to the comparison performed in step e.

According to another embodiment of the method of the fourth aspect of the disclosure, the risk score is computed as a sum of partial thrombosis risk factors, and wherein the partial thrombosis risk factors comprise at least one thrombosis partial risk factor from each parameter extracted from the computational fluid dynamics (CFD) calculations in step b, and at least one thrombosis partial risk factor from clinical data of the patient.

According to another embodiment of the method of the fourth aspect of the disclosure, the thrombosis partial risk factors calculated from the parameters extracted from the CFD calculations are determined by comparing each parameter with one or more risk threshold values for each parameter.

According to another embodiment of the method of the fourth aspect of the disclosure, the clinical data of the patient comprise at least one or more of the following parameters: CHADS2-VASc, HAS-Bleed, left atrium pressure, pulmonary veins pressure, haematocrit levels, haemoglobin levels, and creatinine levels, preferably wherein the clinical data of the patient comprise all of the mentioned parameters.

According to another embodiment of the method of the fourth aspect of the disclosure, the one or more thrombosis partial risk factors calculated from the clinical data of the patient are determined by comparing each parameter comprised in the clinical data of the patient with one or more risk threshold values for each parameter.

According to another embodiment of the method of the fourth aspect of the disclosure, the thrombogenic indexes comprise one or more of the following parameters: hypercoagulability, blood age, and endothelial cell activation potential, preferably wherein the clinical data of the patient comprise all of the mentioned parameters.

According to another embodiment of the method of the fourth aspect of the disclosure, the one or more thrombosis partial risk factors calculated from the thrombogenic indexes of the patient are determined by comparing each thrombogenic index value with one or more risk threshold values for each thrombogenic index.

According to another embodiment of the method of the fourth aspect of the disclosure, the input parameters for the CFD calculations further comprise one or more of the following parameters: CHADS2-VASc, HAS-Bleed, left atrium pressure, pulmonary veins pressure, haemoglobin levels, BNP, C-Protein levels, Troponine levels, eGFR, doppler ultrasound data, ECG data, and creatinine levels, even more preferably wherein the CFD input also comprises all of the mentioned parameters.

According to another embodiment of the method of the fourth aspect of the disclosure, the CFD calculation are performed under simulated movement of the left atrium of the patient, preferably wherein the movement is simulated using an arbitrary Lagrangian-Eulerian method (ALE).

According to another embodiment of the method of the fourth aspect of the disclosure, the simulation of the movement comprises the steps of:
a. comparing the 3D model of the left atrium of the patient with a database of dynamic computed tomography images of moving left atriums from other patients;
b. selecting the most similar dynamic CT image, preferably by using an artificial intelligence algorithm; and
c. using the movements extracted from the selected dynamic CT image, an arbitrary Lagrangian-Eulerian method (ALE), and the CFD input of the patient to simulate the movement of the left atrium of the patient.

According to another embodiment of the method of the fourth aspect of the disclosure, the 3D model of the left atrium of the patient is generated using as input one or more images from one or more imaging techniques from the list comprising: pulsatile ultrasound imaging, Computed Tomography (CT) imaging, Magnetic Resonance Angiography (MRA) imaging, 3D Rotational Angiography (3DRA) imaging, mitral valve pulsatile ultrasound imaging, and pulmonary veins pulsatile ultrasound imaging, preferably wherein the 3D model of the left atrium of the patient is generated using as input images generated using CT imaging, mitral valve pulsatile ultrasound imaging, and pulmonary veins pulsatile ultrasound imaging.

According to another embodiment of the method of the fourth aspect of the disclosure, the 3D model of the left atrium of the patient is automatically generated using neural networks.

According to another embodiment of the method of the fourth aspect of the disclosure, the 3D model of the left atrial appendage occluder is positioned in the 3D model of the left atrial appendage of the patient, wherein the 3D model of the left atrial appendage of the patient is part of the 3D model of the left atrium of the patient, according to at least the diameters of the 3D model of left atrial appendage, anatomical data of the patient and a deployment algorithm, preferably wherein the 3D model of the left atrial appendage occluder is positioned in the 3Dmodel of the left atrium of the patient also according to the clinical data of the patient, more preferably also according to ultrasound imaging.

According to another embodiment of the method of the fourth aspect of the disclosure, the anatomical data of the patient comprise the area of the 3D model of the left atrium of the patient corresponding to the ostium and the Foramen Ovale, preferably wherein the anatomical data of the patient further comprises the landing zone, wherein the landing zone corresponds to a chosen point or subarea comprised in the left atrial appendage of the patient.

According to another embodiment of the method of the fourth aspect of the disclosure, the diameters of the left atrial appendage are calculated according to the 3D model of the left atrial appendage of the patient and a centreline, wherein the centreline is calculated according to the 3D model of the left atrial appendage of the patient and the anatomical data of the patient.

According to another embodiment of the method of the fourth aspect of the disclosure, the deployment algorithm is configured to determine the position of the 3D model of the left atrial appendage occluder in the 3D model of the left atrial appendage of the patient.

According to another embodiment of the method of the fourth aspect of the disclosure, the 3D model of the left atrial appendage occluder is positioned in the 3D model of the left atrial appendage of the patient further according to a database of 3D models of left trial appendage occluders positioned in 3D models of left atrial appendages of other patients.

According to another embodiment of the method of the fourth aspect of the disclosure, the 3D model of the left atrial appendage occluder is selected from a predefined database of left atrial appendage occluders according to the diameters of the 3D model of the left atrial appendage.

According to another embodiment of the method of the fourth aspect of the disclosure, the pressure exerted by the 3D model of the left atrial appendage occluder on the internal walls of the 3D model of the left atrial appendage is calculated through a structural simulation, and if the calculated pressure is greater than a certain threshold, the 3D model of the left atrial appendage occluder is automatically repositioned and the pressure exerted by the 3D model of the left atrial appendage occluder on the internal walls of the 3D model of the left atrial appendage is recalculated, wherein this process can repeat itself until a position is found wherein the pressure exerted is lower than a certain threshold, preferably wherein the threshold is determined by the mechanical properties of the occluder.

According to another embodiment of the method of the fourth aspect of the disclosure, wherein if the computed risk score is above a threshold value, the 3D model of the left atrial appendage occluder is automatically repositioned in the 3D model of the left atrium of the patient until the recomputed risk is below the threshold value.

### Examples

### EXAMPLE 1

Example referred to images 3A and 3B of computational fluid dynamic simulations of a real patient intervention.

### Materials and methods

Two distinct device configurations of the pacifier-type device were proposed for the patient anatomy. The device configurations described a proximal implantation (i.e., surface of the device disk is on the interface plane between the left atrium cavity and the left atrial appendage cavity) and a distal implantation (i.e., surface of the device disk is 4 mm deeper of the interface plane between the left atrium cavity and the left atrial appendage cavity). Both configurations were determined to be a 28 mm size by the maximum (D1) and minimum (D2) diameters of the landing zone (Proximal position: 25 mm and 20 mm for D1 and D2, respectively; Distal position: 28 mm and 22 mm for D1 and D2, respectively), anatomical characteristics (i.e., postero-inferior puncture of the fossa ovalis, type of LAA), and the compression values (Proximal position: 18.52%; Distal position: 27.39%) through steps 3, 4, and, 5 of the general pipeline.

### Results

Figure 3 represents the result of the CFD + ALE simulations (Step 7) performed in both device configurations using the pulsatile echo-Doppler ultrasound at the mitral valve of the patient, blood rheological characteristics, and, the 4D wall LA motion extracted from the most similar patient in the dynamic CT database. Figure 3A describes laminar blood flood behaviour at high velocities (i.e., > 0.2 m/s) on device surface in the proximal position. In the distal position, an eddy blood flow pattern at low velocities (i.e., < 0.2 m/s) is formed in the upper part of the device disk, which is maintained during the rest of the cardiac cycle.

### Conclusion

After the analysis of the several demographic, anatomic and haemodynamic indices comprising the score risk of device-related thrombus, the proximal position should be considered as the optimal configuration in the LAAO procedure for the patient giving the lower score risk of DRT obtained.

## Claims

1. Computer implemented method for *in silico* surgical planification of a left atrial appendage (20) occlusion (LAAO), the method comprising the steps of:
a. generating a 3D model of the left atrium (30) of a patient from medical images of the patient;
b. computing one or more inner diameters of a lumen of the left atrial appendage (LAA) (20), wherein said left atrial appendage (20) is comprised in the 3D model of the left atrium (30) of the patient generated in step a);
c. positioning a 3D model of a left atrial appendage occluder (10) at least partially inside the left atrial appendage (20), further wherein said position is determined according to one or more parameters selected from the list comprising: one or more diameters selected from the one or more diameters calculated in step b), a landing zone position comprised in the 3D model of the left atrial appendage (20), a first compression value of the left atrial appendage occluder (10), and anatomical characteristics extracted from the 3D model of the left atrium (30) of the patient, preferably wherein the anatomical characteristics comprise a shape and position of an ostium and/or a shape and position of an oval fossa; **characterised in that** the method comprises further the steps of:
d. performing a structural simulation to calculate a second compression value of the left atrial appendage occluder (10) from its interaction with the inner walls of the left atrial appendage (20);
e. if the calculated second compression value in step d) is within a range of acceptance, proceeding with step f), and if the second compression value calculated in step d) has a value outside said range of acceptance, continuing by going back to step c) and performing step c) again with the one or more parameters modified, wherein said parameters are preferably a landing zone, the first compression value and/or the one or more diameters;
f. calculating a set of thrombosis risk factors using parameters derived from computational fluid dynamics (CFD) calculations and using medical parameters of the patient;
g. computing a risk score of developing thrombosis related to the left atrial appendage (20) occlusion positioning from step c), wherein said risk score is at least based on the thrombosis risk factors calculated in step f);

2. The computer implemented method according to claim 1, wherein if the risk score computed in step g) is above a risk threshold value, modifying the landing zone position, the first compression value and/or the one or more diameters selected from the one or more diameters calculated in step b), and performing step c) again.

3. The computer implemented method according to claim 1 or 2, wherein if the risk score computed in step g) is above the risk threshold value, displacing the 3D model of the left atrial appendage occluder (10) to a different position according to a database of CFD simulations performed on LAAO from other patients and repeating step d).

4. The computer implemented method according to any of the previous claims, wherein the medical images of the patient are generated from one or more imaging techniques from the list comprising: ultrasound (US) imaging, Transthoracic Echocardiography (TTE), Computed Tomography (CT) imaging, Magnetic Resonance Imaging (MRI) imaging, 3D Rotational Angiography (3DRA) imaging, mitral valve pulsatile ultrasound imaging, and pulmonary veins pulsatile ultrasound imaging; preferably wherein the 3D model of the left atrium (30) of the patient is generated using input images generated using mitral valve pulsatile US imaging and either one of MRI, 3DRA or CT imaging; more preferably wherein the 3D model of the left atrium (30) of the patient is generated using input images generated using mitral valve pulsatile US, pulmonary valve pulsatile US, and either one of MRI, 3DRA or CT imaging.

5. The computer implemented method according to any of the previous claims, wherein the first compression value is based on a volumetric relationship between the volume defined by the unmodified 3D model of the occluder (10) and the volume defined by the 3D model of the occluder (10) when its volume is modified to fit to the dimensions of the internal walls of the 3D model of the left atrium (30) of the patient.

6. The computer implemented method according to any of the previous claims, wherein the position of the LAA occluder determined in step c) is further determined according to clinical data of the patient, preferably wherein the clinical data of the patient comprises one or more of elements from the list comprising: CHADS2-VASc, HAS-Bleed and ultrasound data; more preferably the clinical data further comprises one or more elements from the list comprising: left atrium pressure, haemoglobin levels, BNP, C-Protein levels, Troponine levels, eGFR, ECG data, and creatinine levels; even more preferably wherein the clinical data of the patient comprises all the elements of both lists.

7. The computer implemented method according to any of the previous claims, wherein the position of the LAA occluder determined in step c) is further determined according to at least one or more elements from the list comprising: the landing zone position comprised in the 3D model of the left atrial appendage (20), a database of left atrial appendage occluders (10) positioned in the left atrial appendage (20) of other patients, mechanic and volumetric characteristics of a catheter, wherein said catheter is associated to the positioning of the left atrial appendage occluder (10), and ultrasound images; preferably wherein the position of the LAA occluder is determined according to all the elements of said list.

8. The computer implemented method according to any of the previous claims, wherein the medical parameters of the patient comprise CHADS2-VASc and/or HAS-Bleed.

9. The computer implemented method according to any of the previous claims wherein the thrombosis risk factors derived from CFD calculations comprise one or more of the elements from the list comprising: an average blood velocity in a pulmonary ridge area, a presence and average blood velocity of eddies, and/or stagnated flow in the pulmonary ridge area and a device surface, a particle attachment quantity, and thrombogenic haemodynamic indexes; preferably wherein the thrombosis risk factors derived from CFD calculations comprise all the elements from the list.

10. The computer implemented method according to claim 9, wherein the thrombogenic haemodynamic indexes comprise an endothelial cell activation potential value, and preferably further comprise hypercoagulability and/or blood age values.

11. The computer implemented method according to claim 10, wherein the one or more thrombosis risk factors calculated from the thrombogenic indexes of the patient are determined by comparing each thrombogenic index value with one or more risk threshold values for each thrombogenic index.

12. The computer implemented method according to any of the previous claims, wherein the CFD calculations take as input one or more of physiological parameters from the list comprising pulmonary vein pressure, left atrium pressure, doppler ultrasound data and haematocrit levels, preferably wherein the CFD calculations take as input all the elements of said list.

13. The computer implemented method according to any of the previous claims, wherein the CFD calculations are performed under simulated movement of the left atrium (30) of the patient, preferably wherein the movement is simulated using an Arbitrary Lagrangian-Eulerian method (ALE).

14. The computer implemented method according to claim 13 when depending on claim 12, wherein the simulation of the movement comprises the steps of:
a. comparing the 3D model of the left atrium (30) of the patient with a database of 3D models in motion of the left atrium (30) of other patients obtained from dynamic computed tomography images of moving left atriums (30) from other patients;
b. selecting a most similar 3D model of the left atrium (30) from said database;
c. interpolating the movements extracted from the most similar 3D model of the left atrium (30) selected in step b) to the geometry of the left atrium (30) of the patient; and
d. using the interpolated movements to the left atrium (30) of the patient from step c), an arbitrary Lagrangian-Eulerian method (ALE), and CFD physiological parameters of the patient as input to simulate the movement of the left atrium (30) of the patient.

15. A computer program comprising instructions which, when the program is executed by a computing means, cause the computing means to carry out the method of any of the previous claims.

16. A data processing apparatus comprising a processing unit and a memory unit, wherein the memory unit comprises instructions which, when executed by the processing unit, configures the data processing apparatus to carry out the method of any of claims 1 to 14.

## Patentansprüche

1. Computerimplementiertes Verfahren zur chirurgischen *In-silico* Planifikation einer Okklusion des linken Herzohres (20) (LAAO), wobei das Verfahren die folgenden Schritte umfasst:
a. das Erzeugen eines 3D-Modells des linken Vorhofes (30) eines Patienten aus medizinischen Bildern des Patienten;
b. das Berechnen eines oder mehrerer Innendurchmesser eines Lumens des linken Herzohres (LAA) (20), wobei das genannte linke Herzohr (20) im 3D-Modell des linken Vorhofes (30) des Patienten, welches in Schritt a) erzeugt wurde, umfasst ist;
c. das Positionieren eines 3D-Modells eines linken Herzohr-Okkluders (10) mindestens teilweise innerhalb des linken Herzohres (20), wobei zusätzlich die genannte Position gemäß einem oder mehreren Parametern bestimmt wird, ausgewählt aus der Liste umfassend: einem oder mehreren Durchmessern ausgewählt aus dem einen oder den mehreren Durchmessern, welche in Schritt b) berechnet wurden, einer Landezonenposition, welche im 3D-Modell des linken Herzohres (20) umfasst ist, einem ersten Kompressionswert des linken Herzohr-Okkluders (10), und anatomischen Merkmalen entnommen aus dem 3D-Modell des linken Vorhofes (30) des Patienten, wobei vorzugsweise die anatomischen Merkmale eine Form und Position eines Ostiums und/oder eine Form und Position einer *Fossa ovalis* umfassen;
**dadurch gekennzeichnet, dass** das Verfahren zusätzlich die folgenden Schritte umfasst:
d. das Durchführen eines strukturellen Simulation, um einen zweiten Kompressionswert des linken Herzohr-Okkluders (10) aus dessen Wechselwirkung mit den Innenwänden des linken Herzohres (20) zu berechnen;
e. wenn der berechnete zweite Kompressionswert in Schritt d) innerhalb eines Akzeptanzbereichs liegt, das Fortfahren mit Schritt f), und, wenn der zweite Kompressionswert, welche in Schritt d) berechnet wurde, einen Wert außerhalb dieses Akzeptanzbereichs hat, das Weitermachen indem man zurück zu Schritt c) geht und Schritt c) erneut mit dem einen oder den mehreren modifizierten Paramatern durchführt, wobei die genannten Parameter vorzugsweise eine Landezone, den ersten Kompressionswert und/oder den einen oder die mehreren Durchmesser sind;
f. das Berechnen eines Satzes von Thrombose-Risikofaktoren unter Verwendung von Parametern abgeleitet von Berechnungen der numerischen Strömungsmechanik (CFD) und unter Verwendung von medizinischen Parametern des Patienten;
g. das Berechnen einer Risikobewertung für das Entwickeln vom Thrombose bezüglich der Positionierung der linken Herzohr (20)-Okklusion aus Schritt c), wobei die genannte Risikobewertung mindestens auf den Thrombose-Risikofaktoren, welche in Schritt f) berechnet wurden, basiert.

2. Computerimplementiertes Verfahren nach Anspruch 1, wobei, wenn die Risikobewertung, welche in Schritt g) berechnet wurde, über einem Risiko-Schwellenwert liegt, die Landezonenposition, der erste Kompressionswert und/oder der eine oder die mehreren Durchmesser, welche aus dem einen oder den mehreren Durchmessern ausgewählt werden, welche in Schritt b) berechnet wurden, modifiziert werden und Schritt c) erneut durchgeführt wird.

3. Computerimplementiertes Verfahren nach Anspruch 1 oder 2, wobei, wenn die Risikobewertung, welche in Schritt g) berechnet wurde, über dem Risiko-Schwellenwert liegt, das 3D-Modell des linken Herzohr-Okkluders (10) zu einer unterschiedlichen Position gemäß einer Datenbasis von CFD-Simulationen, welche bei der LAAO von anderen Patienten durchgeführt wurden, verstellt wird und Schritt d) wiederholt wird.

4. Computerimplementiertes Verfahren nach einem der vorhergehenden Ansprüche, wobei die medizinischen Bilder des Patienten aus einer oder mehreren Bildgebungstechniken erzeugt werden, aus der Liste umfassend: Ultraschall (US)-Bildgebung, transthorakale Echokardiographie (TTE), Computertomographie (CT)-Bildgebung, Magnetresonanzbildgebung (MRI), 3D-Rotationsangiographie (3DRA)-Bildgebung, Mitralklappen-Pulsultraschallbildgebung und Lungenvenen-Pulsultraschallbildgebung; wobei vorzugsweise das 3D-Modell des linken Vorhofes (30) des Patienten unter Verwendung von Eingangsbildern erzeugt wird, welche unter Verwendung von Mitralklappen-Pulsultraschallbildgebung und einer von MRI-, 3DRA- oder CT-Bildgebung erzeugt werden; wobei weiter vorzugsweise das 3D-Modell des linken Vorhofes (30) des Patienten unter Verwendung von Eingangsbildern erzeugt wird, welche unter Verwendung von Mitralklappen-Pulsultraschall-, Lungenklappen-Pulsultraschall- und einer von MRI-, 3DRA- oder CT-Bildgebung erzeugt werden.

5. Computerimplementiertes Verfahren nach einem der vorhergehenden Ansprüche, wobei der erste Kompressionswert auf eine, volumetrischen Verhältnis zwischen dem vom nichtmodifizierten 3D-Modell des Okkluders (10) definierten Volumen und dem vom 3D-Modell des Okkluders (10) definierten Volumen basiert, wenn dessen Volumen modifiziert ist, um sich an die Abmessungen der Innenwände des 3D-Modells des linken Vorhofes (30) des Patienten anzupassen.

6. Computerimplementiertes Verfahren nach einem der vorhergehenden Ansprüche, wobei die Position des LAA-Okkluders, welche in Schritt c) bestimmt wurde, zusätzlich gemäß den klinischen Daten des Patienten bestimmt wird, wobei vorzugsweise die klinischen Daten des Patienten ein oder mehrere Elemente umfassen, aus der Liste umfassend: CHADS2-VASc-, HAS-Bleed- und Ultraschalldaten; weiter vorzugsweise die klinischen Daten zusätzlich ein oder mehrere Elemente umfassen, aus der Liste umfassend: den linken Vorhofdruck, Hämoglobinspiegel, BNP, C-Proteinspiegel, Troponinspiegel, eGFR, EKG-Daten und Creatininspiegel; wobei sogar weiter vorzugsweise die klinischen Daten des Patienten alle Elemente beider Listen umfassen.

7. Computerimplementiertes Verfahren nach einem der vorhergehenden Ansprüche, wobei die Position des LAA-Okkluders, welche in Schritt c) bestimmt wurde, zusätzlich gemäß mindestens einem oder mehreren Elementen bestimmt wird, aus der Liste umfassend: die im 3D-Modell des linken Herzohres (20) umfasste Landezonenposition, eine Datenbasis des linken Herzohr-Okkluders (10), welcher im linken Herzohr (20) anderer Patienten positioniert ist, mechanische und volumetrische Merkmale eines Katheters, wobei der genannte Katheter mit der Positionierung des linken Herzohr-Okkluders (10) assoziiert ist, und Ultraschallbilder; wobei vorzugsweise die Position des LAA-Okkluders gemäß allen Elementen der genannten Liste bestimmt wird.

8. Computerimplementiertes Verfahren nach einem der vorhergehenden Ansprüche, wobei die medizinischen Parameter des Patienten CHADS2-VASc und/oder HAS-Bleed umfassen.

9. Computerimplementiertes Verfahren nach einem der vorhergehenden Ansprüche, wobei die Thrombose-Risikofaktoren abgeleitet von CFD-Berechnungen eins oder mehrere der Elemente umfassen, aus der Liste umfassend: eine durchschnittliche Blutgeschwindigkeit in einem Lungenfaltenbereich, ein Vorhandensein und eine durchschnittliche Blutgeschwindigkeit von Wirbeln, und/oder einen gestauten Durchfluss im Lungenfaltenbereich und einer Vorrichtungsoberfläche, eine Partikelanlagerungsmenge und thrombogene hämodynamische Indizes; wobei vorzugsweise die Thrombose-Risikofaktoren abgeleitet von CFD-Berechnungen alle Elemente aus der Liste umfassen.

10. Computerimplementiertes Verfahren nach Anspruch 9, wobei die thrombogenen hämodynamischen Indizes einen Wert für den Endothelzellen-Aktivierungspotential umfassen, und vorzugsweise zusätzlich Hyperkoagulabilitäts- und/oder Blutalterwerte umfassen.

11. Computerimplementiertes Verfahren nach Anspruch 10, wobei der eine oder die mehreren Thrombose-Risikofaktoren berechnet aus den thrombogenen Indizes des Patienten bestimmt werden, indem jeder thrombogene Indexwert mit einem oder mehreren Risiko-Schwellenwerten für jeden thrombogenen Index verglichen wird.

12. Computerimplementiertes Verfahren nach einem der vorhergehenden Ansprüche, wobei die CFD-Berechnungen als Eingang einen oder mehrere der physiologischen Parameter nehmen, aus der Liste umfassend: Lungenvenendruck, linken Vorhofdruck, Doppler-Ultraschalldaten und Hämatokritspiegel, wobei vorzugsweise die CFD-Berechnungen als Eingabe alle Elemente der genannten Liste nehmen.

13. Computerimplementiertes Verfahren nach einem der vorhergehenden Ansprüche, wobei die CFD-Berechnungen unter simulierten Bewegung des linken Vorhofes (30) des Patienten durchgeführt werden, wobei vorzugsweise die Bewegung unter Verwendung einer Arbitrary-Lagrangian-Eulerian-Methode (ALE) simuliert wird.

14. Computerimplementiertes Verfahren nach Anspruch 13, wenn er von Anspruch 12 abhängt, wobei die Simulation der Bewegung die folgenden Schritte umfasst:
a. das Vergleichen des 3D-Modells des linken Vorhofes (30) des Patienten mit einer Datenbasis von 3D-Modellen in Bewegung des linken Vorhofes (30) aus anderen Patienten erhalten aus dynamischen Computertomographiebildern des sich bewegenden linken Vorhofes (30) aus anderen Patienten;
b. das Auswählen eines am meisten ähnlichen 3D-Modells des linken Vorhofes (30) aus der genannten Datenbasis;
c. das Interpolieren der Bewegungen entnommen aus dem am meisten ähnlichen 3D-Modell des linken Vorhofes (30), welches in Schritt b) ausgewählt wurde, mit der Geometrie des linken Vorhofes (30) des Patienten; und
d. das Verwenden der interpolierten Bewegungen des linken Vorhofes (30) des Patienten aus Schritt c), einer Arbitrary-Lagrangian-Eulerian-Methode (ALE) und physiologischer CFD-Parameter des Patienten als Eingabe, um die Bewegung des linken Vorhofes (30) des Patienten zu simulieren.

15. Computerprogramm umfassend Anweisungen, welche, wenn das Programm von einem Computermittel ausgeführt wird, bewirken, dass das Computermittel das Verfahren nach einem der vorhergehenden Ansprüche durchführt.

16. Datenverarbeitungsgerät umfassend eine Verarbeitungseinheit und eine Speichereinheit, wobei die Speichereinheit Anweisungen umfasst, welche, wenn sie von der Verarbeitungseinheit ausgeführt werden, das Datenverarbeitungsgerät dazu einrichten, das Verfahren nach einem der Ansprüche 1 bis 14 durchzuführen.

## Revendications

1. Procédé mis en œuvre par ordinateur pour la planification chirurgicale *in silico* d'une occlusion d'appendice auriculaire gauche (20) (LAAO), le procédé comprenant les étapes consistant à :
a. générer un modèle 3D de l'atrium gauche (30) d'un patient à partir d'images médicales du patient ;
b. calculer un ou plusieurs diamètres internes d'une lumière de l'appendice auriculaire gauche (LAA) (20), dans lequel ledit appendice auriculaire gauche (20) es compris dans le modèle 3D de l'atrium gauche (30) du patient généré dans l'étape a) ;
c. positionner un modèle 3D d'un obturateur d'appendice auriculaire gauche (10) au moins partiellement à l'intérieur de l'appendice auriculaire gauche (20), dans lequel en outre ladite position est déterminée selon un ou plusieurs paramètres choisis dans la liste comprenant : un ou plusieurs diamètres choisis parmi l'un ou plusieurs diamètres calculés dans l'étape b), une position de zone de réception comprise dans le modèle 3D de l'appendice auriculaire gauche (20), une première valeur de compression de l'obturateur d'appendice auriculaire gauche (10), et des caractéristiques anatomiques extraites à partir du modèle 3D de l'atrium gauche (30) du patient, de préférence dans lequel les caractéristiques anatomiques comprennent une forme et une position d'un ostium et/ou une forme et une position d'une fosse ovale ;
**caractérisé en ce que** le procédé comprend en outre les étapes consistant à :
d. réaliser une simulation structurelle pour calculer une deuxième valeur de compression de l'obturateur d'appendice auriculaire gauche (10) à partir de son interaction avec les parois internes de l'appendice auriculaire gauche (20) ;
e. si la deuxième valeur de compression calculée dans l'étape d) se trouve dans un intervalle d'acceptation, continuer avec l'étape f), et si la deuxième valeur de compression calculée dans l'étape d) a une valeur en dehors dudit intervalle d'acceptation, continuer en revenant à l'étape c) et réaliser l'étape c) à nouveau avec l'un ou plusieurs paramètres modifiés, dans lequel lesdits paramètres sont de préférence une zone de réception, la première valeur de compression et/ou l'un ou plusieurs diamètres ;
f. calculer un ensemble de facteurs de risque de thrombose en utilisant des paramètres dérivés à partir de calculs de dynamique de fluides computationnelle (CFD) et en utilisant des paramètres médicaux du patient ;
g. calculer un score de risque de développer une thrombose liée au positionnement de l'occlusion d'appendice auriculaire gauche (20) à partir de l'étape c), dans lequel ledit score de risque est basé, au moins, sur les facteurs de risque de thrombose calculés dans l'étape f).

2. Procédé mis en œuvre par ordinateur selon la revendication 1, dans lequel si le score de risque calculé dans l'étape g) est supérieur à une valeur de seuil de risque, modifier la position de zone de réception, la première valeur de compression et/ou l'un ou plusieurs diamètres choisis parmi l'un ou plusieurs diamètres calculés dans l'étape b), et réaliser l'étape c) à nouveau.

3. Procédé mis en œuvre par ordinateur selon la revendication 1 ou 2, dans lequel si le score de risque calculé dans l'étape g) est supérieur à la valeur de seuil de risque, déplacer le modèle 3D de l'obturateur d'appendice auriculaire gauche (10) vers une position différente selon une base de données de simulations de CFD réalisées sur LAAO à partir d'autres patients et répéter l'étape d).

4. Procédé mis en œuvre par ordinateur selon l'une quelconque des revendications précédentes, dans lequel les images médicales du patient sont générées à partir d'une ou plusieurs techniques d'imagerie de la liste comprenant : imagerie par ultrasons (US), échocardiographie transthoracique (TTE), imagerie par tomodensitométrie (CT), imagerie par résonance magnétique (MRI), imagerie par angiographie rotationnelle 3D (3DRA), imagerie par ultrasons pulsatiles de la valve mitrale, et imagerie par ultrasons pulsatiles des veines pulmonaires ; de préférence dans lequel le modèle 3D de l'atrium gauche (30) du patient est généré en utilisant des images d'entrée générées en utilisant l'imagerie par US pulsatiles de la valve mitrale et l'une quelconque parmi l'imagerie par MRI, 3DRA ou CT ; plus de préférence dans lequel le modèle 3D de l'atrium gauche (30) du patient est généré en utilisant des images d'entrée générées en utilisant des US pulsatiles de la valve mitrale, des US pulsatiles de la valve pulmonaire, et l'une quelconque parmi l'imagerie par MRI, 3DRA ou CT.

5. Procédé mis en œuvre par ordinateur selon l'une quelconque des revendications précédentes, dans lequel la première valeur de compression est basée sur une relation volumétrique entre le volume défini par le modèle 3D no modifié de l'obturateur (10) et le volume défini par le modèle 3D de l'obturateur (10) lorsque son volume est modifié pour s'adapter aux dimensions des parois internes du modèle 3D de l'atrium gauche (30) du patient.

6. Procédé mis en œuvre par ordinateur selon l'une quelconque des revendications précédentes, dans lequel la position de l'obturateur de LAA déterminée dans l'étape c) est déterminée en outre selon des données cliniques du patient, de préférence dans lequel les données cliniques du patient comprennent un ou plusieurs éléments de la liste comprenant : données de CHADS2-VASc, HAS-Bleed et ultrasons ; plus de préférence les données cliniques comprennent en outre un ou plusieurs éléments de la liste comprenant : pression auriculaire gauche, niveaux d'hémoglobine, BNP, niveaux de protéine C, niveaux de troponine, eGFR, données d'ECG, et niveaux de créatinine ; encore plus de préférence dans lequel les données cliniques du patient comprennent tous les éléments des deux listes.

7. Procédé mis en œuvre par ordinateur selon l'une quelconque des revendications précédentes, dans lequel la position de l'obturateur de LAA déterminée dans l'étape c) est déterminée en outre selon au moins un ou plusieurs éléments de la liste comprenant : la position de zone de réception comprise dans le modèle 3D de l'appendice auriculaire gauche (20), une base de données de obturateurs d'appendice auriculaire gauche (10) positionnés dans l'appendice auriculaire gauche (20) d'autres patients, des caractéristiques mécaniques et volumétriques d'un cathéter, dans lequel ledit cathéter est associé au positionnement de l'obturateur d'appendice auriculaire gauche (10), et des images par ultrasons ; de préférence dans lequel la position de l'obturateur de LAA est déterminée selon tous les éléments de ladite liste.

8. Procédé mis en œuvre par ordinateur selon l'une quelconque des revendications précédentes, dans lequel les paramètres médicaux du patient comprennent CHADS2-VASc et/ou HAS-Bleed.

9. Procédé mis en œuvre par ordinateur selon l'une quelconque des revendications précédentes, dans lequel les facteurs de risque de thrombose dérivés à partir de calculs de CFD comprennent un ou plusieurs des éléments de la liste comprenant : une vitesse du sang moyenne dans une zone de crête pulmonaire, une présence et vitesse du sang moyenne de tourbillons, et/ou un écoulement stagnant dans la zone de crête pulmonaire et une surface de dispositif, une quantité de fixation de particules, et des indices hémodynamiques thrombogéniques ; de préférence dans lequel les facteurs de risque de thrombose dérivés à partir de calculs de CFD comprennent tous les éléments de la liste.

10. Procédé mis en œuvre par ordinateur selon la revendication 9, dans lequel les indices hémodynamiques thrombogéniques comprennent une valeur de potentiel d'activation des cellules endothéliales, et de préférence comprennent en outre des valeurs d'hypercoagulabilité et/ou d'âge du sang.

11. Procédé mis en œuvre par ordinateur selon la revendication 10, dans lequel l'un ou plusieurs facteurs de risque de thrombose calculés à partir des indices thrombogéniques du patient sont déterminés par comparaison de chaque valeur d'indice thrombogénique avec un ou plusieurs valeurs de seuil de risque pour chaque indice thrombogénique.

12. Procédé mis en œuvre par ordinateur selon l'une quelconque des revendications précédentes, dans lequel les calculs de CFD prennent en entrée un ou plusieurs paramètres physiologiques de la liste comprenant la pression de veine pulmonaire, la pression auriculaire gauche, des données d'ultrasons par effet Doppler et des niveaux d'hématocrite, de préférence dans lequel les calculs de CFD prennent en entrée tous les éléments de ladite liste.

13. Procédé mis en œuvre par ordinateur selon l'une quelconque des revendications précédentes, dans lequel les calculs de CFD sont réalisés sous un mouvement simulé de l'atrium gauche (30) du patient, de préférence dans lequel le mouvement est simulé en utilisant un procédé arbitraire de Lagrangian-Eulerian (ALE).

14. Procédé mis en œuvre par ordinateur selon la revendication 13 lorsqu'elle dépend de la revendication 12, dans lequel la simulation du mouvement comprend les étapes consistant à :
a. comparer le modèle 3D de l'atrium gauche (30) du patient avec une base de données de modèles 3D en mouvement de l'atrium gauche (30) d'autres patients obtenus à partir d'images de tomodensitométrie dynamique d'atriums gauches (30) en mouvement à partir d'autres patients ;
b. choisir le modèle 3D le plus similaire de l'atrium gauche (30) à partir de ladite base de données ;
c. interpoler les mouvements extraits à partir du modèle 3D le plus similaire de l'atrium gauche (30) choisi dans l'étape b) par rapport à la géométrie de l'atrium gauche (30) du patient ; et
d. utiliser les mouvements interpolés par rapport à l'atrium gauche (30) du patient à partir de l'étape c), un procédé arbitraire de Lagrangian-Eulerian (ALE), et des paramètres physiologiques de CFD du patient en entrée pour simuler le mouvement de l'atrium gauche (30) du patient.

15. Programme informatique comprenant des instructions qui, lorsque le programme est exécuté par des moyens informatiques, amènent les moyens informatiques à mettre en œuvre le procédé selon l'une quelconque des revendications précédentes.

16. Appareil de traitement de données comprenant une unité de traitement et une unité de mémoire, dans lequel l'unité de mémoire comprend des instructions qui, lorsqu'elles sont exécutées par l'unité de traitement, configurent l'appareil de traitement de données pour mettre en œuvre le procédé selon l'une quelconque des revendications 1 à 14.
